# EUROPÄISCHE PATENTANMELDUNG

(11) **EP 1 607 737 A1**
(43) Veröffentlichungstag der Anmeldung: **21.12.2005**
(21) Anmeldenummer: 04014183.0
(22) Anmeldetag: 17.06.2004
(51) Int. Cl.: G01N 21/64, G12B 21/06, G01N 33/543, C12Q 1/68, G01N 33/58, G01N 21/77

(54) **Gerät und Methode zur Detektion und Messung von Nukleinsäuresträngen in Lösung**

(71) Anmelder: Hunziker, Patrick, 4102 Binningen (CH)
(72) Erfinder: Hunziker, Patrick, 4102 Binningen (CH)

(57) **Zusammenfassung**

Die Erfindung beschreibt ein neuartiges Geraet und Methoden, welche den Zweck haben, Nukleinsaeurenstraenge, die faehig sind, an einen komplementaeren Strang zu binden, hoch sensitiv zu detektieren und zu messen. Das Geraet setzt sich typischerweise zusammen aus einem "fluoreszierenden Schalter" (beruhend auf Fluoreszenz-Quenching, Fluroreszenz-Resonanz-Energie-Transfer oder verwandten Methoden), damit verbundenen Nukleinsaeurestraengen, welche an eine transparenten Spitze mit charakteristischer Dimensione in der Nanometerskale gebunden ist, optischen Wellenleitern, und einem hochempfindlichen Photonendetektor. Bei Eintauchen der Spitze in eine Nukleinsaeureloesung binden geloeste und an die Spitze gebundene komplementaere Nukleinsaeurenstraenge aneinander, wodurch die Konformation des gebundenen Nukleinsaeurestrangs modifiziert wird. Dies fuehrt zu einer Aenderung der gegenseitigen Position gebundenen fluoreszierenden Molekuelgruppen und der mit ihnen interagierenden Gruppen, was wiederum eine Aenderung der Fluoreszenzeigenschaften des Komplexes hervorruft, welche bei Exzitation mit Licht eine Aenderung in Intensitaet und Spektrum des emittierten Lichts hervorruft, welche ueber einen Photodetektor gemessen werden kann. Die Hauptanwendungsgebiete der Erfindung sind die Messung von Nukleinsaeurestraengen in Biologie, Forensik, und Medizin.

## Beschreibung

### Beschreibung

Die vorliegende Erfindung beschreibt ein Geraet und Methoden, welche geeignet sind, *Nukleinsaeurenstraenge mit hoher Sensitivitaet und Spezihtaet auf sehr kleinem Raum zu detektieren und zu quantifizieren*. Der Kern der Erfindung ist eine neuartige Kombination einer optisch transparenten Spitze in der Nanometerskala, stark an die Spitze gebundener Nukleinsaeurestraengen die mit funktionellen Molekuelgruppen gekoppelt sind, welche als "fluoreszierende Schalter" fungieren, einer Lichtleiteroptik sowie einem Photonenzaehler. Die Erfindung findet ein breites Anwendungsgebiet in der Medizin und der Biologie: die Detektion und Messung der Konzentration von Nukleinsaeurenstraenge (vor allem Ribonukleinsaeure und Desoxyribonukleinsaeure) wird mit zunehmender Bedeutung der Genomik und der zunehmenden Einsicht in die zentrale Rolle der Genexpression bei physiologischen und pathologischen Veraenderungen immer wichtiger. Zahlreiche Beispiele sind in der Forensik, der Zellbiologie, der Humanphysiologie sowie die Pathophysiologie zu finden. Wie die aeltere Methode der Polymerase Chain Reaction zur Detektion von spezifischen Nukleinsaeurestraengen zeigt, gibt es auch ein grosses industrielles Anwendungspotential in der Pharmaindustrie.

Die verschiedenen Bestandteile des Geraetes sind fuer die Funktionalitaet unabdingbar:
- Die Spitzen ist noetig, um ein sehr kleines Probenvolumen zu erhalten: ein kleinraeumiges Probenvolumen ist wichtig, wenn die *lokale* Konzentration von Nukleinsaeuren, zum Beispiel die unmittelbarer Nachbarschaft einer Zelle oder eines Gewebes fuer die Bestimmung relevant ist um den Typ der gesuchten Zelle zu charakterisieren.
- Die Koppelung der Nukleinsaeurestraenge mit fluoreszierenden Schaltern erlaubt ist fuer den Detektionsmechanismus noetig: Die durch Paarung des oberflaechengebundenen Nukleinsaeurestrangs mit einem im Probenvolumen vorhandenen Nukleinsaeurestrang hervorgerufene Konformationsaenderung fuehrt zu einer Aenderung der sterischen Relation multipler fuer die Fluoreszenz wichtiger funktioneller Molekuelgruppen. Eine Annaeherung zweier derartigen funktioneller Gruppen kann je nach gewaehlter Chemie zu einem Quenching, d.h. zu einer Ausloeschung fuehren oder zu einer Verschiebung der emittierten Wellenlaenge durch Energietransfer "(fluorescence resonance energy transfer).
- Die starke Bindung der derartig funktionalisierten Molekuelgruppen verhindert den Verlust derselben und erlaubt die Wiederverwendung der Probe nach Trennung der beiden Straenge, z.B. durch Loesungen mit hoher Ionendichte.
- Die Verwendung eines Lichtleiters erlaubt, die eigentliche Messung des Signals fern von der Probe durchzufuehren; was die Verwendung von hoechstempfindlichen Photodetektoren wie Single Photon Counters erlaubt; in einer weiteren Anwendung kann die raeumliche Entkoppelung von sensierender Spitze und Messgeraet zu katheterbasierter Diagnose-Tools fuehren. Die Probenspitze kann direkt, ohne Verwendung des Wellenleiters beleuchtet werden und das Fluoreszenzsignal ueber den Wellenleiter zum Photonen Detektor zurueckgefuehrt. In einer Modifikation kann der Lichtwellenleiter kann nur fuer die Zufuhr des excitierenden Lichts benutzt werden und die Emission dann in einem konventionellen Fluoreszenzmikroskop oder einem konfokalen Fluoreszenzmikroskop gemessen werden, was die Separation von excitierendem und emittiertem Licht erleichtert; Dies ist unsere bevorzugte Methode, da damit ein Ein- bzw Auskoppeln des Lichts in den einen Wellenleiter nicht noetig ist und das potentielle praktische Problem der Eigenfluoreszenz gewisser Wellenleiter umgangen wird. Drittens kann das exzitierende Licht mittels Standard-Wellenleitertechnologie anterograd in einen einzelnen Wellenleiter eingekoppelt werden und das emittierte Licht ueber den gleichen Wellenleiter zum Photonen Detektor zurueckgefuehrt werden. Viertens koennen mehrere Lichtleiter fuer Zufuehrung von excitierendem Licht respektive Detektion des emittierten Lichts verwendet werden.

### Herstellung des Geraetes

Optische Spitzen mit einer charakterischen Dimension in der Nanometerskale sind seit der Entwicklung der Optischen Nahfeldmikroskopie in der Schweizzu einer etablierten Technik geworden (*Pohl, D.W., Denk, W., and Dürig, U. (1985). Optical stethoscopy: Imaging with λ*/*20 in Micron and Submicron Integrated Circuit Metrology, Kevin M. Monahan, Editor,* *Proc. SPIE* *565, 56-61.)*. Derartige Spitzen werden oft auf der Basis von Glasfasern hergestellt. Alternativ koennen transparente Spitzen eines Atomic Force Mikroskops verwendet werden, wie sie zum Beispiel aus Siliziumnitrid oder Quartz hergestellt werden. Wir ziehen Glasfasern vor, da dies die Koppelung zum optischen Wellenleiter (typischerweise ebenfalls aus Glasfasern, allenfalls auf Kunststoffbasis) erleichtert, wobei in der bevorzugten Methode sowohl die Spitze als auch der Wellenleiter aus einem Stueck hergestellt werden koennen. Das Abschirmen der Spitze mittels opaquem Material, z.B. durch Aufdampfen von Metallatomen (eine etablierte Technologie), kann das Signal-to-noise Verhaeltnis verbessern. Die Einstrahlung von Streulicht kann zusaetzlich durch optische Abschirmung des Waveguides reduziert werden - derartige Waveguides sind kommerziell erhaeltlich.

Die Herstellung von "fluoreszierenden Schaltern" aus Nukleinsaeuren ist eine junge, aber bereits etablierte Technologie.
Wir bevorzugen die Verwendung von Quantum Dots, welche wie (P.T. Tran et al, Phys.Stat.Sol 2002; 229: 427-432) beschrieben, kovalent an das Substrat gekoppelt werden. Quantum Dots sind aufgrund ihres hohen Signal-zu-Rausch Verhaeltnisses besonders geeignet. Alternativ koennen auf konventionellen Fluorophoren beruhende molekulare beacons, kommerziell erworben werden und enthalten bereits endstaendig Gruppen mit variabler Fluoreszenzaktivitaet in Abhangigkeit der sterischen Konfiguration. Zudem sind Syntheseprokolle veroeffentlicht, z.B. in http://www.molecular-beacons.org/protocol.html" fuer Nukleinsaeurestrange, deren gekoppelte chemische Gruppen ein "Quenching", d.h. eine Daempfung der Fluoreszenz bei raeumlicher Annaeherung zeigen. Applikationen dieser "beacons" in Loesung werden in den US Patenten 6103476 and 6150097 beschrieben. Molekuelgruppen, die einen Fluoreszenz-Resonanz-Energie-Tranfer (FRET) zeigen und damit eine messbare Verschiebung des fluoreszierenden Spektrums, koennen ebenfalls als "Schalterelement" verwendet werden, sie sind publiziert z.B. unter http://www.bme.gatech.edu/groups/bao/beacons.html . Die Bildung von "Excimeren", d.h. von "excited state dimers", die ihr Emissionspektrum in Funktion der raeumlichen Anordnung aendern, kommt als Schaltermechanismus ebenfalls in Frage. Gegenwaertig ziehen wir das "quenching" als Schaltermechanisms vor, da in dieser Technik die meiste Erfahrung besteht.

Die Verwendung von Amino-modifizierten Nukleinsaeuren und ihre Modifikation mittels chemischen Crosslinkern zur Immobilisation auf Oberflaechen ist in der Industrie bekannt, und kann zum Beispiel in *Handbook of Fluorescent Probes and Research Products* 2003 from Molecular Probes Inc, Eugene OR, (http://www.probes.com) gefunden werden. Es bestehen viele etablierte Moeglichkeiten zur Bindung von Molekuelen an eine Oberflaeche. Eine Methode ist die aeltere Technik der Silanisierung der Glasoberflaeche mit Einfuehrung von Aminogruppen gefolgt von der Verwendung eines aminoreaktiven chemischen Crosslinkers. Alternativ kann die Modifikation der Spitze durch Verwendung von t-butyloxycarbonyl wie in (Strother T, Hamers RJ, Smith LM, Nucleic Acids Research 2000; 28:3535-41) erreicht warden. Eine weitere Moeglichkeit ist die Aufdampfung einer sehr duennen Metallsschicht gefolgt von einem Coating der Oberflaeche mit einem self-assembling monolayer (z.B. aufgrund einer Metall-Nichtmetall Bindung wie die Gold-Thiol Bindung oder einer ionischen Bindung).

Die Entwickung von optischen Systemen, die mit optischen Spitzen in der Nanometerskala arbeiten, ist seit der Beschreibung von Pohl (*Pohl, D.W., Denk, W., and Dürig, U. (1985). Optical stethoscopy: Imaging with λ*/*20 in Micron and Submicron Integrated Circuit Metrology, Kevin M*. *Monahan, Editor,* *Proc. SPIE* *565, 56-61*) zur State of the art geworden. Spitzen mit optische Aperturen bis hinunter zu wenigen Nanometern warden in diesen Geraeten verwendet, z.B. beschrieben http://physics.nist.gov/Divisions/Div844/facilities/nsom/nsom.html. Da die Verkleinerung der Apertur mit einer Reduktion der Zahl der beobachteten Molekuele einhergeht bis zum Einzelmolekuel einhergeht, wird das Bindungsverhalten von einem statistischen Phaenomen zu einem On-Off Phaenomen. Waehrend letzteres fuer die ultimative Detektion Einzelmolekuelen eingesetzt werden kann, bevorzugen wir gegenwaertig eine Apertur von ungefaehr 300 Nanometern, da damit eine Multiplizaet von Molekuelen gleichzeitig beobachtet werden kann, und der zeitliche Verlauf der Fluoreszenz (und damit der Bindungsdichte) einen Rueckschluss auf die Konzentration der Molekuele in Loesung zulaesst.

Die hochsensitive Messung von Photonen ist moeglich dank Photonenzaehlern, die eine Effizienz bis 88% fuer die Erfassung von Einzelphotonen besitzen (Takeuchi S, Applied Physics Letter 1999; 74: 1063.) Da pro Fluoreszenzmolekuel bekannterweise mehrere hundert Photonen ausgesandt werden koennen, genuegt diese Sensitivitaet fuer unserer Anwendung bis hinunter zur Detektion von einzelnen Bindungsevents (wobei fuer die Quantifizierung ein partieller Verlust im Verlauf der Uebertragung von Spitze auf Wellenleiter auf Messgeraet beruecksichtigt werden muss). Um eine gute Sensitivitaet zu erreichen, bevorzugen wir Fluorophoren mit niedriger Tendenz zu Bleichung, wie sie kommerziell erhaeltlich sind.

### Anwendung

Die Erfindung ist geeignet fuer alle Anwendungen, bei denen eine Anzahl von Ribonukleinsaeure (RNA-) straengen detektiert und quantifiziert werden soll.

Eine besondere Staerke der Erfindung ist die Moeglichkeit, dies in einem sehr kleinen Probenvolumen zu tun.
Das Anwendungsgebiet dieser Erfindung ist sehr breit.
Infektionen fuehren zur Anwesenheit von bakterialler DNA oder RNA in Koerperfiuessigkeiten, was die Erfindung nuetzlich macht zur Erkennung von Infektionserregern.
Viele Erbkrankheiten sind charakterisiert durch eine abnorme Produktion von Genprodukten. Damit ist die Erfindung nuetzlich zur Erfassung von Vererbungsmustern, Verschwandschaftsbeziehungen und individuellen genetischen Varienten.
Krebszellen zeigen bekannterweise eine abnorme Genexpression, und entsprechend ist es gut bekannt, das Krebszellen aufgrund typischem Gehalt an messenger RNA erkannt werden koennen. Somit eignet sich die Erfindung zur Erkennung von Krebszellen.

### Beschreibung der Figuren

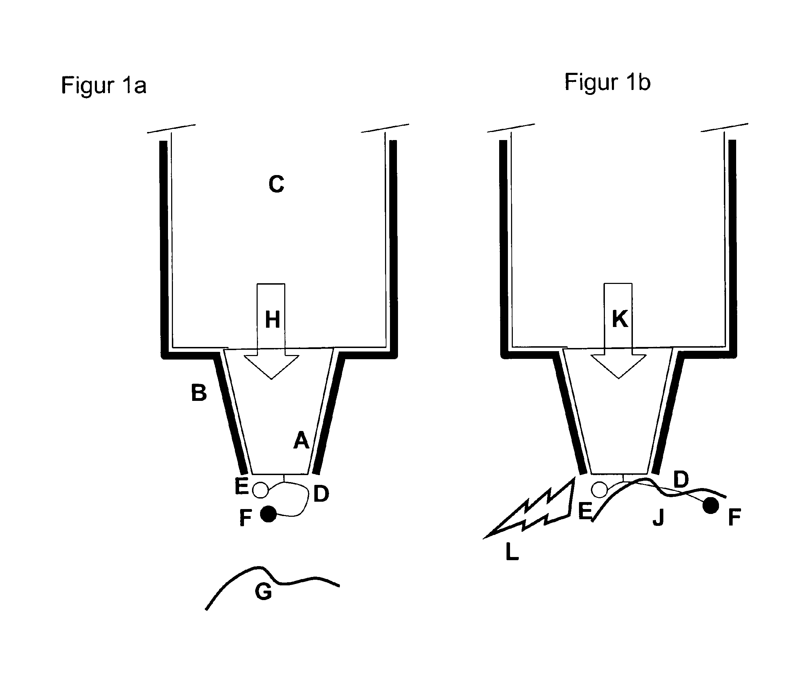

Figur 1 zeigt Aufbau und Funktionsweise der Spitzen A, welche fakultativ mit einer lichtundurchlaessigen Schicht B abgedeckt sind. Die Apertur der Spitze wird bestimmt durch den Durchmesser der mit den Nukleinsaeurestrangen interagierenden Flaeche. D ist ein an die Spitze gebunder Nukleinsaeurestrang, an dessen Enden zwei Molekuelgruppen sitzen, deren Interaktion die Fluoreszenzeigenschaften des Komplexes bestimmen: E ist die fluorescierende Gruppe, F ist eine Molekuelgruppe, welche in Kontakt mit dem Fluorophor die Fluoreszenz ausloescht (ein "quencher"). G ist ein in Loesung befindlicher Nukleinsaeurestrang. Die Einstrahlung excitierenden Lichts H durch den Wellenleiter C loest keine signifikante Fluoreszenz aus, da der Fluorophor in engem Kontakt mit dem Quencher ist. Die Bindung eines in geloesten Komplementaerstrangs J an den gebundenen Strang D fuehrt zu dessen Konformationsaenderung, wodurch Fluorophor E und quencher raeumlich getrennt werden. Jetzt einfallendes excitierendes Licht K fuehrt dadurch zu einer Emission von Fluoreszenzlicht durch Flurophor E, was durch ein Photometer oder ein Fluoreszenzmikroskop gemessen werden kann.

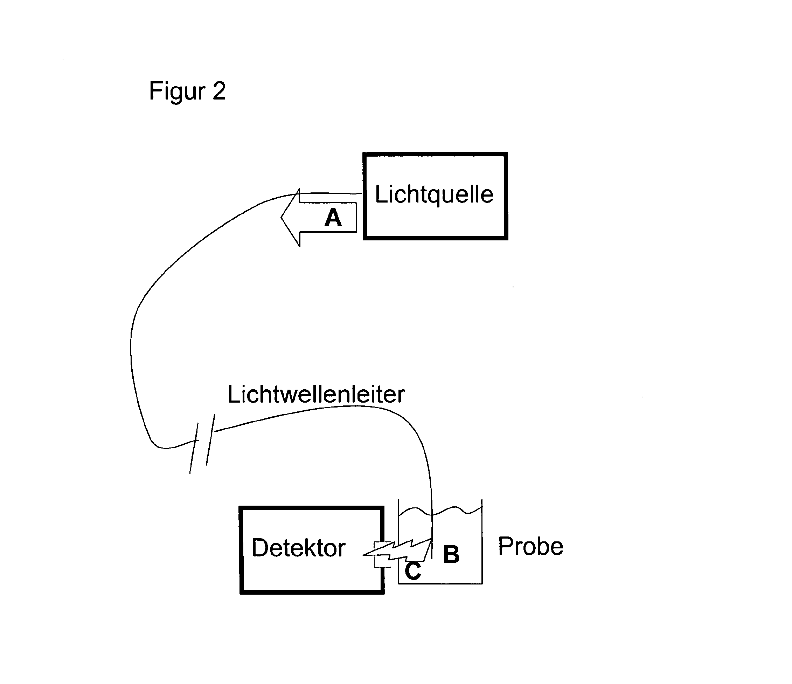

Figur 2 zeigt den grundlegenden Aufbau des Geraetes mit Lichtquelle, Leitung des excitierenden Lichtes A zur Probe, Excitation der Spitze B, vom Fluorophor emittiertes Licht und dessen Messung im Photonendetektor. In alternativem Aufbau kann der Lichtwellenleiter statt fuer die Lichteinleitung zur Lichtherausleitung zum Detektor oder sowohl fuer die Einleitung des exzitierenden wie die Herausleitung des emittierten Lichtes verwendet werden.

## Patentansprüche

1. Ein Geraet zur Messung von einem von: Zahl und Konzentration von Biomolekuelen in Loesung, enthaltend
- eine Anzahl von Probenspitzen, gebaut aus transparentem Material, mit einer charakteristischen Dimension (optische Apertur der Spitze) von 1 bis 1000 Nanometern
- Eine Anzahl von Nukleinsaurenstraengen, gebunden an die genannte Spitze mittels einem von: einer kovalenten Bindung, einem Spacermolekuel, einer ionischen Bindung, einer Metall-Nichtmetall Bindung, wobei der genannte Nukleinsaeurestrang eine >80% Komplementaritaet zur Sequenz des entsprechenden Abschnitts der zu messenden Nukleinsaeure zeigt
- eine Anzahl mit der Nukleinsaeure gekoppelter Quantum Dots und eine Anzahl funktioneller Gruppen, die in raeumlicher Nachbarschaft mit dem Quantum Dot dessen Fluoreszenzeigenschaften aendem.
- Eine Anzahl von optischen Wellenleitern benuetzt fuer das: Leiten des excitierenden Lichts zur Spitze, nicht aber des emittierten Lichts von der Spitze zum Lichtmessgeraet
- Ein Lichtmessgeraet aus der Gruppe: Ein Photonenzaehler, ein Photometer, ein Fluoreszenzmikroskop, ein konfokales Mikroskop, eine Charge-coupled device, ein Halbleiter-Photosensor.

2. Ein Geraet zur Messung von einem von: Zahl und Konzentration von Biomolekuelen in Loesung, enthaltend
- eine Anzahl von Probenspitzen, gebaut aus transparentem Material, mit einer charakteristischen Dimension (Apertur=Interaktionsflaeche der Spitze mit der Probe) von 1 bis 1000 Nanometern
- Eine Anzahl von Nukleinsaurenstraengen, gebunden an die genannte Spitze mittels einem von: einer kovalenten Bindung, einem Spacermolekuel, einer ionischen Bindung, einer Metall-Nichtmetall Bindung, wobei der genannte Nukleinsaeurestrang eine >80% Komplementaritaet zur Sequenz des entsprechenden Abschnitts der zu messenden Nukleinsaeure zeigt
- eine Anzahl der Nukleinsaeure gekoppelter Quantum Dots und eine Anzahl funktioneller Gruppen, die in raeumlicher Nachbarschaft mit dem Quantum Dot dessen Fluoreszenzeigenschaften aendem.
- Eine Anzahl von optischen Wellenleitern benuetzt fuer die Leitung des emittierten Lichts von der Spitze zum Lichtmessgeraet, nicht aber fuer die Leitung des excitierenden Lichts zur Spitze
- Ein Lichtmessgeraet aus der Gruppe: Ein Photonenzaehler, ein Photometer, ein Fluoreszenzmikroskop, ein konfokales Mikroskop, eine Charge-coupled device, ein Halbleiter-Photosensor.

3. Ein Geraet zur Messung von einem von: Zahl und Konzentration von Biomolekuelen in Loesung, enthaltend
- eine Anzahl von Probenspitzen, gebaut aus transparentem Material, mit einer charakteristischen Dimension (Apertur=Interaktionsflaeche der Spitze mit der Probe) von 1 bis 1000 Nanometern
- Eine Anzahl von Nukleinsaurenstraengen, gebunden an die genannte Spitze mittels einem von: einer kovalenten Bindung, einem Spacermolekuel, einer ionischen Bindung, einer Metall-Nichtmetall Bindung, wobei der genannte Nukleinsaeurestrang eine >80% Komplementaritaet zur Sequenz des entsprechenden Abschnitts der zu messenden Nukleinsaeure zeigt
- eine Anzahl der Nukleinsaeure gekoppelter Quantum Dots und eine Anzahl funktioneller Gruppen, die in raeumlicher Nachbarschaft mit dem Quantum Dot dessen Fluoreszenzeigenschaften aendem.
- Eine Anzahl von optischen Wellenleitern benuetzt sowohl fuer das Leiten des excitierenden Lichts zur Spitze als auch fuer die Leitung des emittierten Lichts von der Spitze zum Lichtmessgeraet
- Ein Lichtmessgeraet aus der Gruppe: Ein Photonenzaehler, ein Photometer, ein Fluoreszenzmikroskop, ein konfokales Mikroskop, eine Charge-coupled device, ein Halbleiter-Photosensor.

4. Ein Geraet entsprechend einem von Anspruch 1, Anspruch 2, Anspruch 3, bei welchem die flureszierende Gruppe ein organischer Fluorophor ist.

5. Ein Geraet entsprechend einem von Anspruch 1, Anspruch 2, Anspruch 3, Anspruch 4, bei welchem die fuer die Fluoreszenz verantwortlichen Molekuelgruppen derart gewaehlt sind, dass eines der folgenden Phaenomene bei raeumlicher Konfigurationsaenderung des gebundenen Nukleinsaeurestrangs auftritt: Quenching (Daempfung), Fluoreszenz-Resonanz-Energie-Transfer, Bildung von excited state dimers (Excimers).

6. Eine Methode zur Messung von einem von: der Zahl und der Konzentration von Nukleinsaeurestraengen in einer Loesung, beinhaltend
- die Verwendung einer mit Nukleinsaeurestrang und fluoreszierender Funktionalitaet versehener Spitze gemaess einem von: Anspruch 1, Anspruch 2, Anspruch 3, Anspruch 4, Anspruch 5.
- Die Exposition der genannten Spitze in einer Loesung, die die gesuchten Nukleinsaeurenstraenge enthaelt.
- Die Beleuchtung der genannte Spitze mit excitierendem Licht durch Zufuehrung des Lichts durch einen Wellenleiter
- Das Leiten des von der fluoreszierenden Gruppe emittierten Licht von der genannten Spitze zu einem Detektor entsprechend einem von: Anspruch 1, Anspruch 2, Anspruch 3.

7. Ein Geraet und eine Methode ensprechend einem von: Anspruch 1, Anspruch 2, Anspruch 3, Anspruch 4, Anspruch 5, Anspruch 6, Anspruch 7, Anspruch 8, wobei eine Spitze mit einer charakteristischen Dimension der Spitze 1 Mikrometer bis 100 Mikrometer verwendet wird.

8. Eine Methode entsprechend Anspruch 2, wobei eine Substanz einer der folgenden Klassen gemessen wird: Ribonukleinsaeuren und Desoxyribonukleinsaeuren, und wobei die Probe aus einem der folgenden besteht: Eine Zellkulturfluessigkeit, eine Gewebekulturfluessigkeit, eine Koerperftuessigkeit, eine Gewebefluessigkeit, ein Extrakt eines biologischen Gewebes, das Cytoplasma einer Zelle, eine Bakterienkulturfluessigkeit, eine Pilzkulturfluessigkeit, eine Viruskulturfluessigkeit.

9. Eine Methode entsprechend Anspruch 2, wobei eine Nukleinsaeure bestimmt wird, die gehaeuft auftritt bei folgenden Zustaenden: einem individuellen metabolischen Genotyp, einem individuellen genetisch bedingten Reaktionstyp auf Medikamente, einer Krebserkrankung, einer Kreislauferkrankung.
